(19) Europäisches Patentamt
European Patent Office
Office européen des brevets

(11) Publication number: **0 065 039**
**B1**

(12) **EUROPEAN PATENT SPECIFICATION**

(45) Date of publication of patent specification: **22.08.84**

(21) Application number: **81302009.6**

(22) Date of filing: **07.05.81**

(51) Int. Cl.³: **C 07 F 9/58,** A 01 N 57/16, A 01 N 57/24, A 01 N 57/32 // C07D213/78

(54) **An O-(N-alkoxy-pyridinecarboximidoyl)phosphate, a process for producing the same, and an insecticidal or acaricidal composition containing the same.**

(43) Date of publication of application:
**24.11.82 Bulletin 82/47**

(45) Publication of the grant of the patent:
**22.08.84 Bulletin 84/34**

(84) Designated Contracting States:
**DE FR GB IT**

(56) References cited:
**FR-A-2 163 827**
**GB-A- 836 655**
**US-A-4 054 650**
**US-A-4 076 808**

(73) Proprietor: **ISHIHARA SANGYO KAISHA LTD.**
**No. 3-11, Edobori 1-chome**
**Nishi-ku Osaka-shi Osaka 550 (JP)**

(72) Inventor: **Takahiro, Haga**
**No. 84-7, Hirai-cho**
**Kusatsu-shi Shiga (JP)**
Inventor: **Tadaaki, Toki**
**No. 321-31, Fuke-cho**
**Moriyama-shi Shiga (JP)**
Inventor: **Toru, Koyanagi**
**No. 151-30-3F-410, Ninomaru-cho**
**Mukojima, Fushimi-ku Kyoto-shi Kyoto (JP)**
Inventor: **Osamu, Imai**
**No. 502, Atsumari-cho**
**Kusatsu-shi Shiga (JP)**

(74) Representative: **Roberts, Peter William et al**
**Marks & Clerk Alpha Tower Suffolk Street**
**Queensway**
**Birmingham B1 1TT (GB)**

**EP 0 065 039 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to an O-(N-alkoxy-pyridinecarboximidoyl) phosphate, a process for producing the same, and an insecticide or acaricide containing the same.

An insecticide or acaricide containing an O-(N-alkoxy-carboximidoyl)phosphate is already known; for example, U.S. Patent 4,054,650 discloses that O,O-diethyl-O-(N-methoxy-2-nitrobenzimidoyl)-thionophosphate has an insecticidal or acaricidal activity and U.S. Patent 4,076,808 discloses O,S-dialkyl-O-(N-methoxybenzimidoyl)-dithiophosphate.

It is, however, not known that an O-(N-alkoxy-pyridinecarboximidoyl)phosphate has such insecticidal or acaricidal activity.

It has now been found according to this invention that those compounds represented by the following formula (I) have an insecticidal or acaricidal activity:

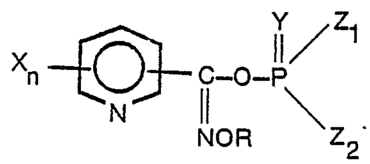

(I)

wherein X is a halogen atom, a lower alkyl group, a lower acyloxy group, a trifluoromethyl group or a nitro group; Y is an oxygen atom or a sulfur atom; R is a lower alkyl group; n is an integer of 0 to 3; and $Z_1$ and $Z_2$ are each a lower alkoxy group, a lower alkylthio group, a phenyl group which may be substituted by a lower alkyl group, a phenoxy group, a haloalkoxy group or a lower alkylamino group.

Preferred compounds are represented by the following formula (II):

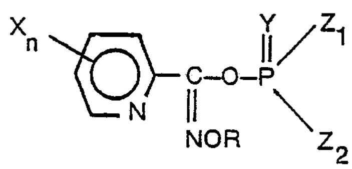

(II)

wherein X, Y, R, n, $Z_1$ and $Z_2$ are the same as defined above.

In particular, the compounds according to this invention, in which the benzene nucleus of the compounds disclosed in U.S. Patent 4,054,650 described above is replaced by a pyridine nucleus, are improved in systemic activity to plant, insecticidal activity or acaricidal activity against insects or acarids including those having a resistivity against the conventional chemicals, as compared to those disclosed in U.S. Patent 4,054,650.

In the definition for the formula (I) above, the halogen atom includes fluorine, chlorine, bromine and iodine; the "alkyl group" in the lower alkyl group, lower alkoxy group, lower alkylthio grtoup and lower alkylamino group means an alkyl group having 1 to 4 carbon atoms, such as methyl, ethyl, n-propyl, isopropyl, n-butyl, sec-butyl, tert-butyl and isobutyl; the lower acyloxy group includes acetyloxy and propionyloxy; and the haloalkoxy group includes trifluoroethoxy.

Of the compounds represented by the formula (I), those represented by the following formula (III) are preferred.

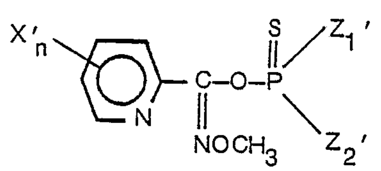

(III)

wherein X' is a halogen atom, a lower alkyl group, a trifluoromethyl group or a nitro group; $Z_1'$ and $Z_2'$ are each a lower alkoxy group, a lower alkylthio group or a phenyl group; and n is the same as defined in the formula (I) above.

More preferred are those represented by the formula (IV) below:

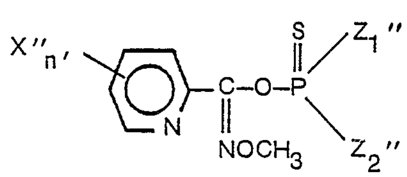

(IV)

wherein X″ is a halogen atom or a trifluoromethyl group; n′ is an integer of 0 to 2; and $Z_1''$ and $Z_2''$ are each a lower alkoxy group or a phenyl group.

In the above formulae (I), (III) and (IV), when the pyridine nucleus has a substituent represented by $X_n$, $X'_n$ or $X''_n$, wherein n or n′ is 2 or 3, the substituents may be the same or different.

The compound represented by the formula (I) of this invention is usually prepared by reacting an N-alkoxy-pyridinecarboximidic acid represented by the formula (A):

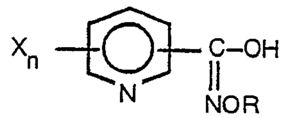

(A)

wherein X, R and n are the same as described above, and a known phosphoric halide represented by the formula (B):

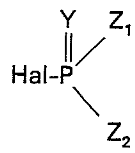

(B)

wherein Hal is a halogen atom, and Y, $Z_1$ and $Z_2$ are the same as described above, in the presence of an alkaline material.

Examples of the alkaline materials which can be used include sodium hydroxide, potassium hydroxide, sodium carbonate, potassium carbonate and potassium methylate.

The above reaction is preferably carried out in an inert organic solvent. Examples of the inert organic solvents which can be used are nitriles such as acetonitrile or propionitrile, ethers such as dioxane or diethyl ether, ketones such as acetone, methyl ethyl ketone or methyl isobutyl ketone, and aprotic solvents such as tetrahydrofuran.

The reaction temperature is 0° to 150°C, preferably 20° to 90°C, and the reaction time is 1 to 30 hours, preferably 2 to 10 hours. The reaction is usually carried out at atmospheric pressure.

The N-alkoxy-pyridinecarboximidic acid represented by the formula (A) can easily be prepared by, for example, (1) a method in which pyridine-hydroxamic acid is reacted with an alkylating agent, such as an alkyl halide, or a dialkylsulfuric acid, and (2) a method in which a picolinic acid derivative, such as a halide or ester of picolinic acid, is reacted with an O-alkoxyamine.

Synthesis Example 1

Synthesis of O,O-Diethyl-O-(N-methoxy-pyridine-2-carboximidoyl)thionophosphate

(A) *Preparation of N-Methoxy-pyridine-2-carboximidic acid*

6.8 g (0.08 mol) of an O-methylhydroxylamine hydrochloric acid salt was dissolved in 20 ml of water, and 22.4 g (0.16 mol) of potassium carbonate dissolved in 20 ml of water was dropwise added thereto at 0°C. 14.2 g (0.08 mol) of a picolinyl chloride hydrochloric acid salt was further added thereto, and the resulting solution was stirred at 0° to 10°C for 4 hours. The reaction product thus obtained was extracted with methylene chloride several times. The organic phase was dried over anhydrous sodium sulfate to obtain 8.4 g of N-methoxy-pyridine-2-carboximidic acid.

(B) *Preparation of the desired product*

8.4 g (0.055 mol) of N-methoxy-pyridine-2-carboximidic acid was dissolved in 40 ml of acetonitrile, and 9 g (0.065 mol) of a powder of potassium carbonate was added thereto. The mixture was heated to 50°C with stirring, and 10.4 g (0.055 mol) of O,O-diethylthionophosphoric acid diester chloride was dropwise added thereto. The resulting mixture was then further reacted at 50°C for 24 hours with stirring. The reaction product was allowed to cool and poured into a suitable amount of water. An oily phase was extracted with methylene chloride. The extracted phase was washed successively with a saturated sodium chloride aqueous solution, a 2N-sodium hydroxide solution and water, and then dried over anhydrous sodium sulfate. After the drying, the solvent was distilled off under reduced pressure, and the residue was purified by silica gel column chromatography using an n-hexane-ethyl acetate mixed solvent as an eluant, to obtain 8.2 g of the desired product ($n_D^{32.8}$ 1.5199).

Synthesis Example 2

Synthesis of O-Ethyl-O-(N-methoxy-pyridine-2-carboximidoyl)-N-sec-butyl Phosphoramidothioate

3 g (0.02 mol) of N-methoxy-pyridine-2-carboximidic acid was dissolved in 40 ml of acetonitrile, and 3.2 g (0.02 mol) of potassium carbonate was added thereto. The mixture was heated to 50°C with stirring, and after dropwise addition of 4.3 g (0.02 mol) of O-ethyl-N-sec-butyl thionophosphoric acid amide ester chloride, the reaction was conducted at 50°C for 24 hours while stirring. The reaction

product was allowed to cool and poured into a suitable amount of water. An oily phase was extracted with methylene chloride. The extracted phase was washed successively with a saturated sodium chloride aqueous solution, a 2N-sodium hydroxide solution and water, and then dried over anhydrous sodium sulfate. After the drying, the solvent was distilled off under reduced pressure, and the residue was purified by silica gel column chromatography using an n-hexane-ethyl acetate mixed solvent as an eluant to obtain 4.2 g of the desired product ($n_D^{20.0}$ 1.5410).

Synthesis Example 3

Synthesis of O-Ethyl-O-(N-ethoxy-pyridine-2-carboximidoyl)phenyl Phosphonothioate

(A) *Preparation of N-Ethoxy-pyridine-2-carboximidic acid*

7 g (0.05 mol) of pyridine-2-hydroxamic acid and 1.4 g (0.05 mol) of sodium hydride were dissolved in 100 ml of ethanol, and 8 g (0.05 mol) of ethyl iodide was gradually dropwise added thereto with stirring. After the dropwise addition, the reaction solution was stirred for 2 hours under reflux condition. After completion of the reaction, the ethanol was distilled off under reduced pressure. To the residue were added suitable amounts of methylene chloride and water, whereby the desired product was extracted with the methylene chloride. The extract was washed with a saturated sodium chloride aqueous solution, and dried over anhydrous sodium sulfate. After the drying, the solvent was distilled off under reduced pressure to obtain 8.2 g of the desired product.

(B) *Preparation of the final product*

2 g (0.012 mol of N-ethoxy-pyridine-2-carboximidic acid was dissolved in 40 ml of acetonitrile, and 2 g (0.014 mol) of a powder of potassium carbonate was added thereto. The mixture was heated to 50°C while stirring, and after dropwise addition of 2.7 g (0.012 mol) of O-ethylphenylthionophosphoric acid chloride, the reaction was conducted at 50°C for 8 hours with stirring. The reaction product was allowed to cool, poured into a suitable amount of water, and an oily phase was extracted with methylene chloride. The extracted phase was washed successively with a saturated sodium chloride aqueous solution, a 2N-sodium hydroxide aqueous solution and water, and dried over anhydrous sodium sulfate. After the drying, the solvent was distilled off under reduced pressure, and the residue was purified by silica gel column chromatography using an n-hexane-ethyl acetate mixed solvent as an eluant to obtain 3.1 g of the desired final product ($n_D^{23.4}$ 1.5710).

Examples of the compounds of the formula (I) of this invention are shown in Table 1.

TABLE 1

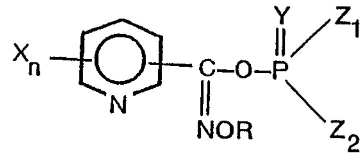

| Compound No. | Pyridyl Moiety | Y | R | $Z_1$ | $Z_2$ | Physical Properties |
|---|---|---|---|---|---|---|
| 1 | 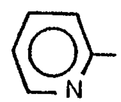 | S | $CH_3$ | $-OCH_3$ | $-OCH_3$ | $n_D^{22.5}$ 1.5489 |
| 2 | ,, | S | ,, | $-OC_2H_5$ | $-OC_2H_5$ | $n_D^{32.8}$ 1.5199 |
| 3 | ,, | O | ,, | $-OC_2H_5$ | $-SC_3H_7$-n | $n_D^{20.5}$ 1.5310 |
| 4 | ,, | O | ,, | $-OC_3H_7$-n | $-OC_3H_7$-n | $n_D^{23.0}$ 1.4715 |
| 5 | ,, | O | ,, | $-OC_2H_5$ | 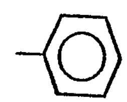 | $n_D^{19.8}$ 1.5515 |
| 6 | ,, | S | ,, | $-OC_2H_5$ | ,, | $n_D^{18.3}$ 1.5947 |
| 7 | ,, | O | ,, | 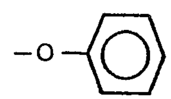 | 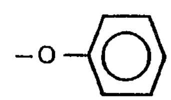 | $n_D^{15.5}$ 1.5980 |

TABLE 1 (Continued)

| Compound No. | Pyridyl Moiety | Y | R | $Z_1$ | $Z_2$ | Physical Properties |
|---|---|---|---|---|---|---|
| 8 | 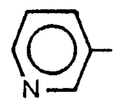 | S | $CH_3$ | $-OC_2H_5$ | $-OC_2H_5$ | $n_D^{22.5}$ 1.5345 |
| 9 | 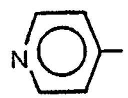 | S | ,, | $-OC_2H_5$ | $-OC_2H_5$ | $n_D^{20.3}$ 1.5315 |
| 10 | 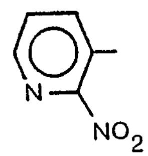 | S | ,, | $-OC_2H_5$ | $-OC_2H_5$ | $n_D^{23.0}$ 1.5340 |
| 11 | 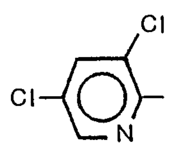 | S | ,, | $-OC_2H_5$ | $-OC_2H_5$ | $n_D^{18.3}$ 1.5490 |
| 12 | 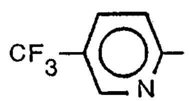 | S | ,, | $-OC_2H_5$ | $-OC_2H_5$ | $n_D^{22.0}$ 1.5065 |
| 13 | 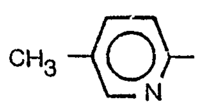 | S | ,, | $-OC_2H_5$ | $-OC_2H_5$ | $n_D^{22.5}$ 1.5321 |

TABLE 1 (Continued)

| Compound No. | Pyridyl Moiety | Y | R | $Z_1$ | $Z_2$ | Physical Properties |
|---|---|---|---|---|---|---|
| 14 | 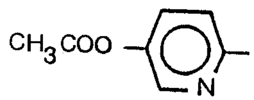 | S | $CH_3$ | $-OC_2H_5$ | $-OC_2H_5$ | $n_D^{15.5}$ 1.5329 |
| 15 | 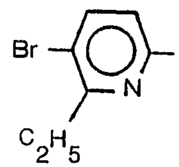 | S | ,, | $-OC_2H_5$ | $-OC_2H_5$ | $n_D^{15.5}$ 1.5898 |
| 16 | 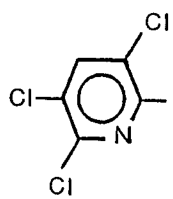 | S | ,, | $-OC_2H_5$ | $-OC_2H_5$ | $n_D^{16.0}$ 1.5080 |
| 17 | 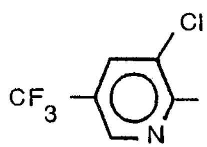 | S | ,, | $-OC_2H_5$ | $-OC_2H_5$ | $n_D^{16.0}$ 1.5054 |
| 18 | 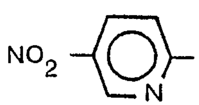 | S | ,, | $-OC_2H_5$ | $-OC_2H_5$ | $n_D^{20.0}$ 1.5210 |
| 19 | 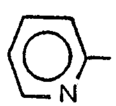 | S | ,, | $-OC_2H_5$ | $NHCHC_2H_5$<br>\|<br>$CH_3$ | $n_D^{20.0}$ 1.5410 |

TABLE 1 (Continued)

| Compound No. | Pyridyl Moiety | Y | R | $Z_1$ | $Z_2$ | Physical Properties |
|---|---|---|---|---|---|---|
| 20 | 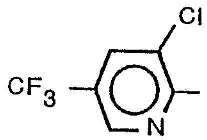 | S | $CH_3$ | $-OC_2H_5$ | 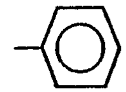 | $n_D^{17.8}$ 1.5479 |
| 21 | 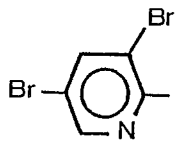 | S | ,, | $-OC_2H_5$ | $-OC_2H_5$ | $n_D^{15.0}$ 1.5876 |
| 22 | 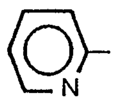 | S | ,, | $-OCH_3$ | 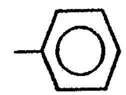 | $n_D^{15.5}$ 1.6180 |
| 23 | ,, | S | ,, | $-OC_3H_7$-n | ,, | $n_D^{15.5}$ 1.6019 |
| 24 | ,, | S | ,, | $-SC_2H_5$ | ,, | $n_D^{16.8}$ 1.6300 |
| 25 | 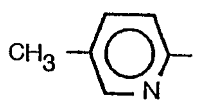 | S | ,, | $-OC_2H_5$ | ,, | $n_D^{19.0}$ 1.5880 |
| 26 | 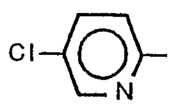 | S | ,, | $-OCH_3$ | ,, | $n_D^{15.1}$ 1.6074 |

TABLE 1 (Continued)

| Compound No. | Pyridyl Moiety | Y | R | $Z_1$ | $Z_2$ | Physical Properties |
|---|---|---|---|---|---|---|
| 27 | 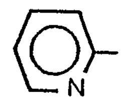 | S | $C_2H_5$ | $-OC_2H_5$ | 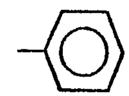 | $n_D^{23.4}$ 1.5710 |
| 28 | ,, | S | $C_2H_5$ | $-OC_2H_5$ | $-OC_2H_5$ | $n_D^{23.4}$ 1.5175 |
| 29 | ,, | S | $C_3H_7$-n | $-OC_2H_5$ | 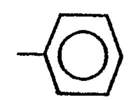 | $n_D^{27.5}$ 1.5666 |
| 30 | ,, | S | $C_3H_7$-i | $-OC_2H_5$ | ,, | $n_D^{25.5}$ 1.5650 |
| 31 | ,, | S | $CH_3$ | $-OC_2H_5$ | 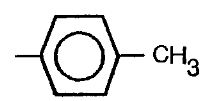 | $n_D^{14.4}$ 1.5998 |
| 32 | ,, | S | $CH_3$ | $-OCH_2CF_3$ | 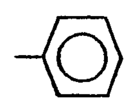 | $n_D^{17.6}$ 1.5548 |

Test Example 1

Diamondback Moth (*Plutella xylostella*)-Control Test

20 parts by weight of an active ingredient, 60 parts by weight of xylene and 20 parts by weight of a mixture of a polyoxyethylene phenyl phenol derivative, a polyoxyethylene sorbitan alkylate and an alkylaryl sulfonate were uniformly mixed to prepare an emulsifiable concentrate which was then diluted with water to provide an emulsion having a concentration of the active ingredient of 200 ppm.

A section of cabbage leaf was immersed in the emulsion for about 10 seconds, taken out therefrom and dried in air. A wetted filter paper was placed on a Petri dish (diameter: 9 cm), and the section was placed thereon. Some 2nd to 3rd instar larvae of diamondback moth were released on the lamina and allowed to stand in an illuminated covered thermostat maintained at 28°C. Two days after the release of larvae, the alive and dead of the larvae were evaluated. The percent mortality was calculated by the following equation:

$$\text{Percent Mortality} = \frac{\text{Number of Dead Larvae}}{\text{Number of Released Larvae}} \times 100\ (\%)$$

The results obtained are shown in Table 2—1.

TABLE 2—1

| Compound No. | Percent Mortality (%) |
|---|---|
| 1 | 100 |
| 5 | 100 |
| 10 | 100 |
| 11 | 100 |
| 12 | 100 |
| 13 | 100 |
| 17 | 100 |
| 21 | 100 |
| 22 | 100 |
| 23 | 100 |
| 24 | 50 |
| 25 | 90 |
| 26 | 100 |
| 27 | 100 |
| 28 | 100 |
| 31 | 90 |
| 32 | 100 |

Comparison in activity between the compounds of this invention and O,O-diethyl-O-(N-methoxy-2-nitrobenzimidoyl)thionophosphate disclosed in U.S. Patent 4,054,650 (hereinafter referred to as "comparison compound") was made in the same manner as in Test Example 1 except that the concentration of the active ingredient in each emulsion was reduced. The results obtained are shown in Table 2—2.

TABLE 2—2

| Compound No. | Percent Mortality (%) Concentration of Active Ingredient 100 ppm | 25 ppm | 6.25 ppm |
|---|---|---|---|
| 1 | 100 | 100 | 100 |
| 2 | 100 | 80 | 90 |
| 6 | 80 | 70 | 70 |
| Comparison Compound | 0 | 0 | 0 |

Test Example 2

Housefly (*Musca domestica*)-Control Test

A powdery feed (produced by Oriental Yeast Industry Co., Ltd.) as a culture medium for larvae of housefly, bran and the same emulsion as prepared in Test Example 1 which had been adjusted to a predetermined concentration of the active ingredient were mixed in weight ratio of 1:1:2, and the mixture was placed in a cup (diameter: 7 cm, height: 4 cm). Some 2nd to 3rd instar larvae of housefly were released into the cup, and the cup was then covered with gauze. Twelve days after the release of larvae, the alive and dead were evaluated. The percent mortality was measured in the same manner as in Test Example 1. The results obtained are shown in Table 3—1.

TABLE 3—1

| Compound No. | Percent Mortality (%) Concentration of Active Ingredient 40 ppm | 10 ppm | 25 ppm |
|---|---|---|---|
| 2 | 100 | 100 | 100 |
| 11 | 100 | 100 | 100 |
| 12 | 100 | 100 | 100 |

Comparison in activity between the compounds of this invention and the comparison compound was made in the same manner as in Test Example 2 except that the concentration of active ingredient in the emulsion was reduced. The results obtained are shown in Table 3—2.

TABLE 3—2

| Compound No. | Percent Mortality (at 0.6 ppm) (%) |
|---|---|
| 1 | 90 |
| 2 | 100 |
| Comparison Compound | 40 |

Test Example 3

Azuki Bean Weevil (*Callosobruchus chinensis*)-Control Test

The same emulsifiable concentrate as prepared in Test Example 1 was adjusted to provide an emulsion having a predetermined concentration of the active ingredient. 1 ml of the emulsion was uniformly attached to the inside bottom surface of a 9 cm Petri dish and dried in air to provide a Petri dish with the predetermined amount of the active ingredient attached in the form of a film on the inner surface thereof. After releasing 15 adult azuki bean weevils thereinto, the dish was covered and placed

for 24 hours in a thermostat maintained at 25°C. Thereafter, the alive and dead were evaluated to thereby measure the percent mortality in the same manner as in Test Example 1. The results obtained are shown in Table 4—1.

TABLE 4—1

| Compound No. | Percent Mortality (%) Amount of Active Ingredient 100 $\mu$g/dish | 50 $\mu$g/dish | 25 $\mu$g/dish |
|---|---|---|---|
| 1 | 100 | 100 | 100 |
| 2 | 100 | 100 | 100 |
| 3 | 100 | 100 | 100 |
| 9 | 100 | 100 | 100 |
| 10 | 100 | 100 | 100 |
| 11 | 100 | 100 | 100 |
| 12 | 100 | 100 | 100 |
| 13 | 100 | 100 | 100 |
| 14 | 100 | 100 | 100 |
| 16 | 100 | 100 | 100 |
| 17 | 100 | 100 | 100 |
| 18 | 100 | 100 | 100 |
| 20 | 100 | 100 | 100 |
| 21 | 100 | 100 | 100 |
| 22 | 100 | 100 | 100 |
| 26 | 100 | 100 | 100 |
| 27 | 100 | 100 | 100 |
| 28 | 100 | 100 | 100 |

Comparison in activity between the compounds of this invention and the comparison compound was made in the same manner as in Test Example 3 except that the concentration of the active ingredient in the emulsion was reduced. The results obtained are shown in Table 4—2.

TABLE 4—2

| Compound No. | Percent Mortality (at 3.125 μg/dish) |
|---|---|
| | (%) |
| 1 | 90 |
| 2 | 100 |
| 6 | 60 |
| 11 | 100 |
| 17 | 100 |
| Comparison Compound | 0 |

Test Example 4

Green Rice Leafhopper (*Nephotettix cincticeps*)- and Smaller Brown Planthopper (*Delphacodes striatella*)-Control Test

A wettable powder as prepared in Formulation Example 3 set forth hereinbelow was adjusted to a concentration of active ingredient of 100 ppm to prepare a solution. Only the root part of rice plant seedling was immersed in the solution for 48 hours. After completely washing the root part with water, the stem part was covered with defatted cotton and placed in a test tube. Some 3—4 instar larvae of green rice leafhopper and smaller brown planthopper were released into the test tube, and the test tube was then covered with gauze. After 48 hours, the alive and dead of the larvae were evaluated to thereby measure the percent mortality in the same manner as in Test Example 1. The results obtained are shown in Table 5—1.

TABLE 5—1

| Compound No. | Percent Mortality<br>Green Rice Leafhopper | <br>Smaller Brown Planthopper |
|---|---|---|
| | (%) | (%) |
| 1 | 100 | 100 |
| 2 | 100 | 100 |
| 4 | 100 | 100 |
| 6 | 100 | 100 |
| 11 | 100 | 100 |
| 12 | 100 | 100 |
| 18 | 100 | — |
| 19 | 100 | — |
| 20 | 100 | — |
| 21 | 100 | — |
| 22 | 100 | — |
| 23 | 100 | — |
| 24 | 40 | — |
| 25 | 100 | — |
| 26 | 100 | — |
| 26 | 100 | — |
| 27 | 100 | 100 |
| 28 | 100 | 100 |
| 31 | 100 | — |
| 32 | 100 | — |

Comparison in activity between the compounds of this invention and the comparison compound was made in the same manner as in Test Example 4 except that the concentration of active ingredient in the solution was adjusted to 25 ppm. The results obtained are shown in Table 5—2.

TABLE 5—2

| Compound No. | Percent Mortality<br>Green Rice Leafhopper | <br>Smaller Brown Planthopper |
|---|---|---|
| | (%) | (%) |
| 1 | 60 | 100 |
| 2 | 100 | 100 |
| 6 | — | 100 |
| 10 | 60 | 100 |
| 17 | 50 | 100 |
| Comparison Compound | 0 | 50 |

Test Example 5

Carmine Mite (*Tetranychus telarius*)-Control Test

A kidney bean young plant whose leaves were cut leaving one primary leaf was planted in an ice cream cup, inoculated with about 30 of larvae and adults of carmine mite, and then immersed for 10 seconds in the emulsion as used in Test Example 1 and adjusted to a concentration of the active ingredient of 200 ppm. After drying in air, the plant was placed in a thermostat with illumination and maintained at 28°C. Three days later, the alive and dead of the larvae and adults were evaluated to thereby measure the percent mortality in the same manner as in Test Example 1. The results obtained are shown in Table 6—1.

TABLE 6—1

| Compound No. | Percent Mortality (%) |
|---|---|
| 1 | 100 |
| 2 | 100 |
| 4 | 100 |
| 6 | 100 |
| 11 | 100 |
| 12 | 100 |
| 18 | 100 |
| 19 | 50 |
| 20 | 100 |
| 21 | 100 |
| 22 | 100 |
| 23 | 100 |
| 24 | 100 |
| 25 | 90 |
| 26 | 100 |
| 31 | 70 |
| 32 | 100 |

Comparison in activity between the compounds of this invention and the comparison compound was made in the same manner as in Test Example 5 except that the concentration of active ingredient in the emulsion was adjusted to 25 ppm. The results obtained are shown in Table 6—2.

TABLE 6—2

| Compound No. | Percent Mortality (%) |
|---|---|
| 1 | 100 |
| 6 | 100 |
| 21 | 90 |
| 22 | 100 |
| 23 | 90 |
| Comparison Compound | 50 |

Test Example 6

Green Peach Aphid (*Myzus persicae*)-Control Test

On an eggplant seedling (about 20 cm high) which was cultivated in a 1/3,500 are pot and on which a number of green peach aphidae were parasitic was sprayed an emulsion prepared by adjusting

an emulsifiable concentrate as prepared in Formulation Example 2 set forth hereinbelow to a concentration of the active ingredient of 50 ppm, in an amount of 20 ml per the pot. The eggplant seedling was held in a greenhouse/three days later, the reduction in the number of green peach aphidae was observed. The results obtained are shown in Table 7. The survival rate was evaluated on the following scale:

1. Nearly equal to the untreated plot.
2. Fair survival although a reduction is observed.
3. Fair reduction.
4. Slight survival.
5. No survival.

TABLE 7

| Compound No. | Survival Rate |
|---|---|
| 1 | 5 |
| 2 | 5 |
| 4 | 5 |
| 6 | 5 |
| 11 | 5 |
| 12 | 5 |
| 18 | 4 |
| 20 | 4 |
| 21 | 5 |
| 22 | 5 |
| 23 | 4 |
| 24 | 5 |
| 31 | 5 |

O-(N-alkoxy-pyridinecarboximidoyl)phosphates of the formula (I) are effective against various harmful insects and acarids.

Examples of such harmful insects against which the compounds of this invention are effective are Hemiptera such as aphidae [e.g., cotton aphid (*Aphis gossypii*), cabbage aphid (*Brevicoryne brassicae*), green peach apid (*Myzus persicae*), etc.], coccidae [e.g., *Lesanium cerni, Saissetis eleas,* California red scale (*Aonidielle aurantii*), *Aspridiotue hederae,* etc.], deltocephalidae [e.g., green rice leafhopper (*Nephotettix cincticeps*), etc.], delphacidae [e.g., smaller brown planthopper (*Delphacodes striatella*), brown planthopper (*Nilaparvata lugens*), etc.]; Lepidoptera such as diamondback moth (*Plutella xylostella, Heliothis spp.,* army worm (*Mamestra brassicae*), tobacco cutwarm (*Spodoptera litura*), etc.; Diptera such as housefly (*Musca domestica*), *Culex pipiens pallens*, etc.; Coleoptera such as azuki bean weevil (*Callosobruchus chinensis*), 28-spotted lady beetle (*Epilachna vigintioctopunctata*).

Examples of harmful acarids against of which the compounds of this invention are also effective are two-spotted spider mite (*Tetranychus urticae*), citrus red mite (*Panonychus citri*).

The O-(N-alkoxy-pyridinecarboximidoyl)phosphates as described above exhibit an excellent insecticidal activity, particularly against sucking insects, and an excellent acaricidal effect against acarids of *Tetranychus* which are resistant against various known acaricides.

The insecticidal or acaricidal composition of this invention is usually used in a concentration of active ingredient of 1 to 10,000 ppm, preferably 20 to 2,000 ppm. In the case of aquatic insects, they can be controlled by spraying the composition in the above concentration range and, therefore, the concentration range in water is effective even below the above-specified range.

When the compounds of this invention are used as active ingredients of the insecticidal or acaricidal composition, it is possible to formulate into various forms, such as dust, wettable powder, emulsifiable concentrate, inert emulsion, oil solution and aerosol preparation, with agriculturally effective amounts of agriculturally acceptable adjuvants. The composition can be applied with or without diluting in suitable concentrations.

Suitable examples of the adjuvants which can be used include powdery carriers such as talc, kaolin, bentonite, diatomaceous earth, silicon dioxide, clay and starch, liquid diluents such as water, xylene, toluene, dimethyl sulfoxide, dimethylformamide, acetonitrile, and alcohols, emulsifiers, dispersing agents, spreaders.

A suitable concentration of the active ingredient in the insecticidal or acaricidal composition is usually 5 to 80% by weight for the case of emulsifiable concentrate, 0.5 to 30% by weight for the case of dust and 5 to 60% by weight for the case of wettable powder, respectively.

It is also possible to combine with other agricultural ingredients such as other insecticides, acaricides and plant growth regulators. Sometimes, synergistic effects are found. For example, organophosphoric acid ester compounds, carbamate compounds, dithio (or thio) carbamate compounds, organochloro compounds, dinitro compounds, organic sulfur or metal compounds, antibiotics, substituted diphenyl ether compounds, urea compounds, triazine compounds, benzoyl urea compounds and pyrethroid compounds can be exemplified. More specifically, benzoyl urea compounds such as N-(2,6-difluorobenzoyl)-N′-(p-chlorophenyl)urea, and pyrethroid compounds such as $\alpha$-cyano-3-phenoxybenzyl 2-(4-chlorphenyl)isovalerate can be exemplified.

Formulation examples containing the compound of this invention are shown below.

Formulation Example 1

| | parts by weight |
|---|---|
| O,O-Dimethyl-O-(N-methoxy-pyridine-2-carboximidoyl)-thionophosphate | 20 |
| Xylene | 60 |
| Sorpol 2806B (trade name for a mixture of polyoxyethylenephenyl phenol derivatives, polyoxyethylenealkyl aryl ether, polyoxyethylene-sorbitan alkylate and alkyl arylsulfate, produced by Toho Chemical Industry Co., Ltd.) | 20 |

These ingredients were uniformly mixed and dissolved to provide an emulsifiable concentrate.

Formulation Example 2

| | parts by weight |
|---|---|
| O,O-Diethyl-O-(N-methoxy-pyridine-2-carboximidoyl)thionophosphate | 70 |
| Xylene | 20 |
| Polyoxyethylene Alkylphenyl Ether | 10 |

These ingredients were uniformly mixed and dissolved to provide an emulsifiable concentrate.

Formulation Example 3

| | parts by weight |
|---|---|
| O,O-Di-n-propyl-O-(N-methoxy-pyridine-2-carboximidoyl)phosphate | 30 |
| Sodium Dodecylsulfate | 2 |
| Sodium Dinaphthylmethanesulfonate | 3 |
| Fine Silicon Dioxide ($SiO_2 \cdot nH_2O$) | 20 |
| Diatomaceous Earth | 45 |

These ingredients were uniformly mixed to provide a wettable powder.

Formulation Example 4

| | parts by weight |
|---|---|
| O,O-Diethyl-O-(N-methoxy-5-trifluoromethyl-pyridine-2-carboximidoyl)thionophosphate | 5 |
| Talc | 95 |

These ingredients were uniformly pulverized and mixed to provide a dust.

Formulation Example 5

| | parts by weight |
|---|---|
| O,O-Diethyl-O-(N-methoxy-3,5-dichloropyridine-2-carboximidoyl)thionophosphate | 5 |
| Sodium Ligninsulfonate | 3 |
| Sodium Alkylbenzenesulfonate | 2 |
| Bentonite | 30 |
| Talc | 60 |

These ingredients were mixed with a suitable amount of water necessary for granulation and then granulated.

**Claims**

1. An O-(N-alkoxy-pyridinecarboximidoyl)phosphate represented by the following formula (I):

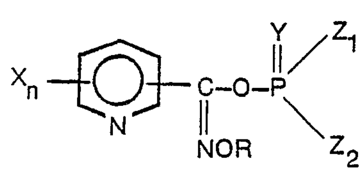

(I)

wherein X is a halogen atom, a lower alkyl group, a lower acyloxy group, a trifluoromethyl group or a nitro group; Y is an oxygen atom or a sulfur atom; R is a lower alkyl group; n is an integer of 0 to 3; and $Z_1$ and $Z_2$ are each a lower alkoxy group, a lower alkylthio group, a phenyl group which may be substituted with a lower alkyl group, a phenoxy group, a haloalkoxy group or a lower alkylamino group.

2. A compound of Claim 1, which is represented by the following formula (II):

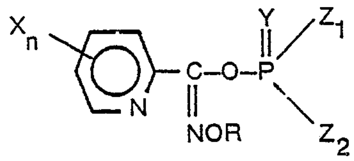

(II)

wherein X, Y, R, n, $Z_1$ and $Z_2$ are the same as defined in Claim 1.

3. A compound of Claim 1, which is represented by the following formula (III):

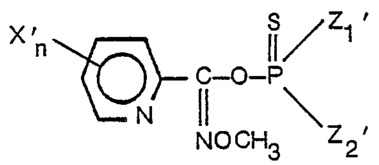

(III)

wherein X′ is a halogen atom, a lower alkyl group, a trifluoromethyl group or a nitro group; $Z_1'$ and $Z_2'$ are each a lower alkoxy group, a lower alkylthio group or a phenyl group; and n is the same as defined in Claim 1.

4. A compound of Claim 1, which is represented by the following formula (IV):

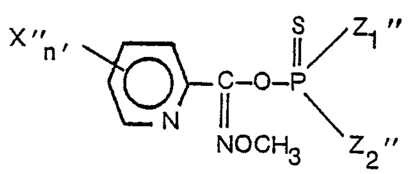

(IV)

wherein X″ is a halogen atom or a trifluoromethyl group; n′ is an integer of 0 to 2; and $Z_1''$ and $Z_2''$ are each a lower alkoxy group or a phenyl group.

5. O-ethyl-O-(N-methoxy-pyridine-2-carboximidoyl)phenyl phosphonothionate.

6. O,O-dimethyl-O-(N-methoxy-pyridine-2-carboximidoyl)thionophosphate.

7. O,O-diethyl-O-(N-methoxy-pyridine-2-carboximidoyl)thionophosphate.

8. O,O-diethyl-O-(N-methoxy-3,5-dichloropyridine-2-carboximidoyl)thionophosphate.

9. O,O-diethyl-O-(N-methoxy-3-chloro-5-trifluoromethylpyridine-2-carboximidoyl)thionophosphate.

10. O-methyl-O-(N-methoxy-pyridine-2-carboximidoyl)phenyl phosphonothionate.

11. An insecticidal or acaricidal composition comprising an insectidally or acaricidally effective amount of an O-(N-alkoxy-pyridinecarboxamidoyl)phosphate having the formula (I) according to Claim 1 as an active ingredient and an agriculturally acceptable adjuvant.

12. An insecticidal or acaricidal composition according to Claim 11, which comprises 0.5 to 80 parts by weight of the O-(N-alkoxy-pyridinecarboximidoyl)phosphate having the formula (I) and 20 to 99.5 parts by weight of the agriculturally acceptable adjuvant.

13. An insecticidal or acaricidal composition according to Claim 11, which comprises 0.5 to 80 parts by weight of the O-(N-methoxy-pyridine-2-carboximidoyl)thionophosphate having the formula (IV) and 20 to 99.5 parts by weight of the agriculturally acceptable adjuvant.

14. An insecticidal or acaricidal composition according to Claim 11, wherein the active ingredient is O-ethyl-O-(N-methoxy-pyridine-2-carboximidoyl)phenyl phosphonothionate.

15. An insecticidal or acaricidal composition according to Claim 11, wherein the active ingredient is O,O-dimethyl-O-(N-methoxy-pyridine-2-carboximidoyl)thionophosphate.

16. An insecticidal or acaracidal composition according to Claim 11, wherein the active ingredient is O,O-diethyl-O-(N-methoxy-pyridine-2-carboximidoyl)thionophosphate.

17. An insecticidal or acaricidal composition according to Claim 11, wherein the active ingredient is O,O-diethyl-O-(N-methoxy-3,5-dichloropyridine-2-carboximidoyl)thionophosphate.

18. An insecticidal or acaricidal composition according to Claim 11, wherein the active ingredient is O,O-diethyl-O-(N-methoxy-3-chloro-5-trifluoromethylpyridine-2-carboximidoyl)thionophosphate.

19. An insecticidal or acaricidal composition according to Claim 11, wherein the active ingredient is O-methyl-O-(N-methoxy-pyridine-2-carboximidoyl)phenyl phosphonothionate.

20. A process for producing an O-(N-alkoxy-pyridinecarboximidoyl)phosphate having the formula (I):

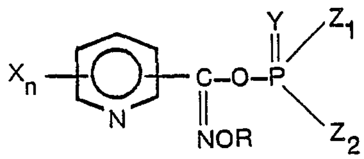

(I)

wherein X is a halogen atom, a lower alkyl group, a lower acyloxy group, a trifluoromethyl group or a nitro group; Y is an oxygen atom or a sulfur atom; R is a lower alkyl group; n is an integer of 0 to 3; and $Z_1$ and $Z_2$ are each a lower alkoxy group, a lower alkylthio group, a phenyl group, which may be substituted with a lower alkyl group, a phenoxy group, a haloalkoxy group or a lower alkylamino group, which comprises reacting an N-alkoxy-pyridinecarboximidic acid having the formula (A):

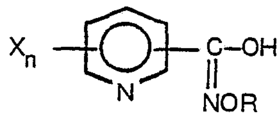

(A)

wherein X, R and n are the same as defined hereinbefore, with a phosphoric halide having the formula (B):

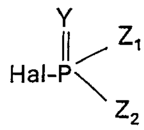

(B)

wherein Hal is a halogen atom, and Y, $Z_1$ and $Z_2$ are the same as defined hereinbefore, in the presence of an alkaline material.

21. A process according to Claim 20, wherein the reaction is carried out in the presence of an organic solvent.

22. A process according to Claim 20, wherein the reaction is carried out at a temperature of 0°C to 150°C.

**Patentansprüche**

1. Ein O-(N-Alkoxy-pyridincarboximidoyl)phosphat der allgemeinen Formel (I)

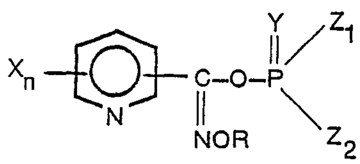

(I)

worin X ein Halogenatom, eine Niedrigalkylgruppe, eine Niedrigacyloxygruppe, eine Trifluormethylgruppe oder eine Nitrogruppe; Y ein Sauerstoffatom oder ein Schwefelatom; R eine Niedrigalkylgruppe; n eine ganze Zahl von 0 bis 3; und $Z_1$ und $Z_2$ jeweils eine Niedrigalkoxygruppe, eine Niedrigalkylthiogruppe, eine Phenylgruppe, die durch eine Niedrigalkylgruppe, eine Phenoxygruppe, eine Haloalkoxygruppe oder eine Niedrigalkylaminogruppe substituiert sein kann, bedeuten.

2. Eine Verbindung gemäss Anspruch 1 der allgemeinen Formel (II)

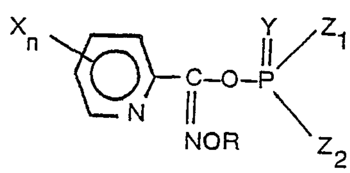

(II)

worin X, Y, R, n, $Z_1$ und $Z_2$ die in Anspruch 1 angegebenen Bedeutungen haben.

3. Eine Verbindung gemäss Anspruch 1, der allgemeinen Formel (III)

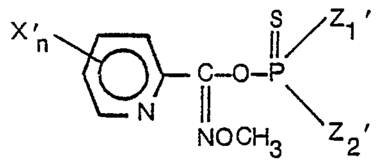

(III)

worin X′ ein Halogenatom, eine Niedrigalkylgruppe, eine Trifluormethylgruppe oder eine Nitrogruppe, $Z_1'$ und $Z_2'$ jeweils eine Niedrigalkoxygruppe, eine Niedrigalkylthiogruppe oder eine Phenylgruppe bedeuten und n die gleiche Bedeutung wie in Anspruch 1 hat.

4. Eine Verbindung gemäss Anspruch 1, der allgemeinen Formel (IV)

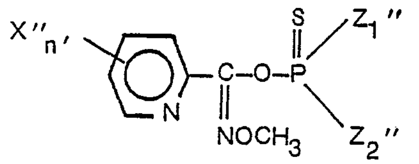

(IV)

worin X″ ein Halogenatom oder eine Trifluormethylgruppe, n eine ganze Zahl von 0 bis 2 und $Z_1''$ und $Z_2''$ jeweils eine Niedrigalkoxygruppe oder eine Phenylgruppe bedeuten.

5. O-Ethyl-O-(N-methoxy-pyridin-2-carboximidoyl)phenylphosphonothionat.

6. O,O-Dimethyl-O-(N-methoxy-pyridin-2-carboximidoyl)thionophosphat.

7. O,O-Diethyl-O-(N-methoxy-pyridin-2-carboximidoyl)thionophosphat.

8. O,O-Diethyl-O-(N-methoxy-3,5-dichloropyridin-2-carboximidoyl)thionophosphat.

9. O,O-Diethyl-O-(N-methoxy-3-chloro-5-trifluoromethylpyridin-2-carboximidoyl)thionophosphat.

10. O-Methyl-O-(N-methoxy-pyridin-2-carboximidoyl)phenylphosphonothionat.

11. Zusammensetzung gegen Insekten oder Milben, dadurch gekennzeichnet, dass sie eine gegen Insekten oder Milben wirksame Menge eine O-(N-Alkoxy-pyridincarboximidoyl)phosphats der allgemeinen Formel (I) gemäss Anspruch 1 als aktiven Bestandteil und einen landwirtschaftlich annehmbaren Träger enthält.

12. Zusammensetzung gegen Insekten oder Milben gemäss Anspruch 11, dadurch gekennzeichnet, dass sie 0,5 bis 80 Gew.-Teile des O-(N-Alkoxy-pyridincarboximidoyl)phosphats der allgemeinen Formel (I) und 20 bis 99,5 Gew.-Teile des landwirtschaftlich annehmbaren Trägers enthält.

13. Zusammensetzung gegen Insekten und Milben gemäss Anspruch 11, dadurch gekennzeichnet, dass sie 0,5 bis 80 Gew.-Teile des O-(N-Methoxy-pyridin-2-carboximidoyl)thionophosphats der Formel (IV) und 20 bis 99,5 Gew.-Teile des landwirtschaftlich annehmbaren Trägers enthält.

14. Zusammensetzung gegen Insekten oder Milben gemäss Anspruch 11, dadurch gekennzeichnet, dass der aktive Bestandteil O-Ethyl-O-(N-methoxy-pyridin-2-carboximidoyl)phenylphosphonothionat ist.

15. Zusammensetzung gegen Insekten oder Milben gemäss Anspruch 11, dadurch gekennzeichnet, dass der aktive Bestandteil O,O-Dimethyl-O-(N-methoxy-pyridin-2-carboximidoyl)thionophosphat ist.

16. Zusammensetzung gegen Insekten oder Milben gemäss Anspruch 11, dadurch gekennzeichnet, dass der aktive Bestandteil O,O-Diethyl-O-(N-methoxy-pyridin-2-carboximidoyl)thionophosphat ist.

17. Zusammensetzung gegen Insekten oder Milben gemäss Anspruch 11, dadurch gekennzeichnet, dass der aktive Bestandteil O,O-Diethyl-O-(N-methoxy-3,5-dichloropyridin-2-carboximidoyl)thionophosphat ist.

18. Zusammensetzung gegen Insekten oder Milben gemäss Anspruch 11, dadurch gekennzeichnet, dass der aktive Bestandteil O,O-Diethyl-O-(N-methoxy-3-chloro-5-trifluoromethylpyridin-2-carboximidoyl)thionophosphat ist.

19. Zusammensetzung gegen Insekten oder Milben gemäss Anspruch 11, dadurch gekennzeichnet, dass der aktive Bestandteil O-Methyl-O-(N-methoxy-pyridin-2-carboximidoyl)phenylphosphonothionat ist.

20. Verfahren zur Herstellung eines O-N-Alkoxy-pyridincarboximidoyl)phosphats der Formel (I)

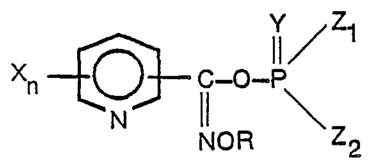

(I)

worin X ein Halogenatom, eine Niedrigalkylgruppe, eine Niedrigacyloxygruppe, eine Trifluormethylgruppe oder eine Nitrogruppe; Y ein Sauerstoffatom oder ein Schwefelatom; R eine Niedrigalkylgruppe; n eine ganze Zahl von 0 bis 3 und $Z_1$ und $Z_2$ jeweils eine Niedrigalkoxygruppe, eine Niedrigalkylthiogruppe, eine Phenylgruppe, die durch eine Niedrigalkylgruppe oder eine Phenoxygruppe, eine Haloalkoxygruppe oder eine Niedrigalkylaminogruppe substituiert sein kann, bedeuten, dadurch gekennzeichnet, dass man eine N-Alkoxy-pyridincarboximidsäure der Formel (A)

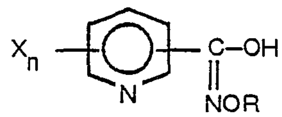

(A)

worin X, R und n die vorher angegebenen Bedeutungen haben, mit einem Phosphorsäurehalogenid der Formel (B)

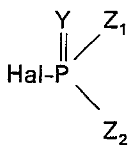

(B)

worin Hal ein Halogenatom und Y, $Z_1$ und $Z_2$ die vorher angegebenen Bedeutungen haben, in Gegenwart eines alkalischen Materials umsetzt.

21. Verfahren gemäss Anspruch 20, dadurch gekennzeichnet, dass man die Umsetzung in Gegenwart eines organischen Lösungsmittels durchführt.

22. Verfahren gemäs Anspruch 20, dadurch gekennzeichnet, dass man die Umsetzung bei einer Temperatur von 0 bis 150°C durchführt.

**Revendications**

1. Un O-(N-alcoxy-pyridinecarboximidoyl)-phosphate représenté par la formule (I) suivante:

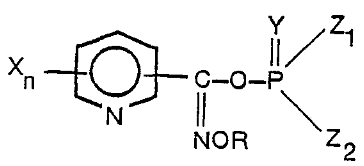

dans laquelle X est un atome d'halogène, un groupe alkyle inférieur, un groupe acyloxy inférieur, un groupe trifluorométhyle ou un groupe nitro; Y est un atome d'oxygène ou un atome de soufre; R est un groupe alkyle inférieur; n est un entier de 0 à 3; et $Z_1$ et $Z_2$ sont chacun un groupe alcoxy inférieur, un groupe alkylthio inférieur, un groupe phényle qui peut être substitué par un groupe alkyle inférieur, un groupe phénoxy, un groupe halogénoalcoxy ou un groupe alkylamino inférieur.

2. Un composé selon la revendication 1, qui est représenté par la formule (II) suivante:

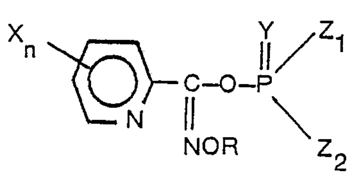

(II)

dans laquelle X, Y, R, n, $Z_1$ et $Z_2$ sont tels que définis à la revendication 1.

3. Un composé selon la revendication 1, qui est représenté par la formule (III) suivante:

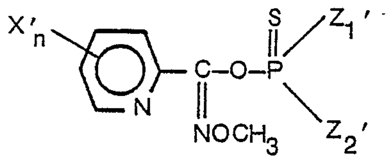

(III)

dans laquelle X′ est un atome d'halogène, un groupe alkyle inférieur, un groupe trifluorométhyle ou un groupe nitro; $Z_1'$ et $Z_2'$ sont chacun un groupe alcoxy inférieur, un groupe alkylthio inférieur ou un groupe phényle; et n est tel que défini dans la revendication 1.

4. Un composé selon la revendication 1, qui est représenté par la formule (IV) suivante:

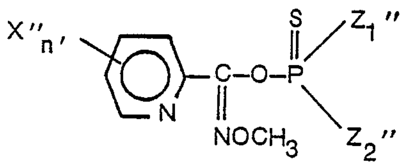

(IV)

dans laquelle X″ est un atome d'halogène ou un groupe trifluorométhyle, n′ est un entier de 0 à 2; et $Z_1''$ et $Z_2''$ sont chacun un groupe alcoxy inférieur ou un groupe phényle.

5. Le (N-méthoxy-pyridine-2-carboximidoyl)-phénylphosphonothionate de O-éthyle.

6. Le O-(N-méthoxy-pyridine-2-carboximidoyl)-thionophosphate de O,O-diméthyle.

7. Le O-(N-méthoxy-pyridine-2-carboximidoyl)-thionophosphate de O,O-diéthyle.

8. Le O-(N-méthoxy-3,5-dichloropyridine-2-carboximidoyl)-thionophosphate de O,O-diéthyle.

9. Le O-(N-méthoxy-3-chloro-5-trifluorométhylpyridine-2-carboximidoyl)-thionophosphate de O,O-diéthyle.

10. Le O-(N-méthoxy-pyridine-2-carboximidoyl)-phénylphosphonothionate de O-méthyle.

11. Une composition insecticide ou acaricide comprenant une quantité efficace comme insecticide ou acaricide d'un O-(N-alcoxy-pyridinecarboximidoyl)-phosphate répondant à la formule (I) selon la revendication 1 comme ingrédient actif et un adjuvant acceptable en agriculture.

12. Une composition insecticide ou acaricide selon la revendication 11, qui consiste en 0,5 à 80 parties en poids du O-(N-alcoxy-pyridinecarboximidoyl)-phosphate de formule (I) et 20 à 99,5 parties en poids de l'adjuvant acceptable en agriculture.

13. Une composition insecticide ou acaricide selon la revendication 11, qui consiste en 0,5 à 80 parties en poids du du O-(N-méthoxy-pyridine-2-carboximidoyl)-thionophosphate de formule (IV) et 20 à 99,5 parties en poids de l'adjuvant acceptable en agriculture.

14. Une composition insecticide ou acaricide selon la revendication 11, dans laquelle l'ingrédient actif est le O-(N-méthoxy-pyridine-2-carboximidoyl)-phénylphosphonothionate de O-éthyle.

15. Une composition insecticide ou acaricide selon la revendication 11, dans laquelle l'ingrédient actif est le O-(N-méthoxy-pyridine-2-carboximidoyl)-thionophosphate de O,O-diéthyle.

16. Une composition insecticide ou acaricide selon la revendication 11, dans laquelle l'ingrédient actif est le O-(N-méthoxy-pyridine-2-carboximidoyl)-thionophosphate de O,O-diéthyle.

17. Une composition insecticide ou acaricide selon la revendication 11, dans laquelle l'ingrédient actif est le O-(N-méthoxy-3,5-dichloropyridine-2-carboximidoyl)thionophosphate de O,O-diéthyle.

18. Une composition insecticide ou acaricide selon la revendication 11, dans laquelle l'ingrédient actif est le O-(N-méthoxy-3-chloro-5-trifluorométhylpyridine-2-carboximidoyl)thionophosphate de O,O-diéthyle.

19. Une composition insecticide ou acaricide selon la revendication 11, dans laquelle l'ingrédient actif est le O-(N-méthoxy-pyridine-2-carboximidoyl)-phénylphosphonothionate de O-méthyle.

20. Un procédé de fabrication d'un O-(N-alcoxy-pyridine-carboximidoyl)-phosphate de formule (I):

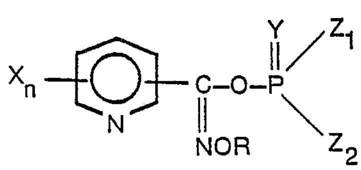

(I)

dans laquelle X est un atome d'halogène, un groupe alkyle inférieur, un groupe acyloxy inférieur, un groupe trifluorométhyle ou un groupe nitro; Y est un atome d'oxygène ou de soufre; R est un groupe

alkyle inférieur; n est un entier de 0 à 3 et $Z_1$ et $Z_2$ sont chacun un groupe alcoxy inférieur, un groupe alkylthio inférieur, un groupe phényle qui peut être substitué par un groupe alkyle inférieur, un groupe phénoxy, un groupe halogénoalcoxy ou un groupe alkylamino inférieur, qui consiste à faire réagir un acide N-(alcoxy-pyridinecarboximidique de formule (A):

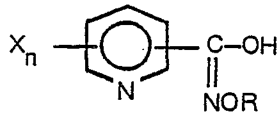

(A)

dans laquelle X, R et n sont tels que définis ci-dessus avec un halogénure d'acide phosphorique de formule (B):

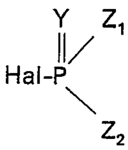

(B)

dans laquelle Hal est un atome d'halogène et Y, $Z_1$ et $Z_2$ sont tels que définis ci-dessus, en présence d'une substance alcaline.

21. Un procédé selon la revendication 20, dans lequel la réaction est effectuée en présence d'un solvant organique.

22. Un procédé selon la revendication 20, dans lequel la réaction est effectuée à une température de 0 à 150°C.